# EUROPEAN PATENT APPLICATION

(11) **EP 0 888 764 A1**
(43) Date of publication of application: **07.01.1999**
(21) Application number: 97110736.2
(22) Date of filing: 01.07.1997
(51) Int. Cl.: A61F 13/15, A61F 13/56

(54) **Use of a handling aid for a body applied disposable absorbent article**

(71) Applicant: THE PROCTER & GAMBLE COMPANY, Cincinnati, Ohio 45202 (US)
(72) Inventor: Wierlacher, Stefan Alois, 65122 Pescara (IT)
(74) Representative: Hirsch, Uwe Thomas

(57) **Abstract**

The invention provides a use of a means for holding and applying directly to a user's body a disposable absorbent article having a body facing surface and a garment facing surface, a longitudinal symmetry plane, a front end edge and a rear end edge, and comprising a liquid pervious topsheet, a backsheet joined to said topsheet and an absorbent core intermediate the backsheet and the topsheet. The means are located on the garment facing surface of the absorbent article, and are oriented transversely. The means are used by inserting at least one finger in the means, by manipulating the article into a position directly on the body, and by preferably forming the article into a tridimensional shape while applying it to the body.

## Description

### FIELD OF THE INVENTION

The present invention relates to disposable absorbent articles. Disposable absorbent articles are considered to be absorbent devices designed to be worn externally of the body by a user and to receive fluids discharged from the body. In particular the present invention relates to disposable absorbent sanitary napkins, catamenials, incontinence inserts, and pantiliners intended for direct application to the wearer's body, and provided with means for said application and, preferably, for a successive removal of the article, and to the use of said means for said application.

### BACKGROUND OF THE INVENTION

In their basic form, disposable absorbent articles comprise an absorbent core interposed between a pervious body-contacting element (alternatively referred to as a topsheet or an overwrap) and an impervious protective barrier (alternatively referred to as a backsheet). The absorbent element is, of course, intended to receive and contain the fluids discharged from the body. The body-contacting element is intended to provide comfortable and dry-feeling contact with body surfaces while allowing free passage of fluids therethrough into the absorbent element. The protective barrier is intended to prevent the fluids which are expelled or which escape from the absorbent element from soiling the user's garments.

Most known disposable absorbent articles are intended to be applied to the panty, and typically fixed to it by an adhesive, before wearing the panty with the applied absorbent article, and this does not facilitate a good fit with the body anatomy since the absorbent article has first to adapt to the surface of the garment, and also owing to differences in wearing habits and in panty styles. Major disadvantages of known disposable absorbent articles intended to be worn externally of the body after being previously applied to the panty, e.g. leakage, wet/dirty feeling, discomfort, are in fact related to the poor body fit achieved by these articles.

It has therefore been recognized that direct application of a disposable absorbent article to the body of the wearer can largely improve the performance of the product, since the product itself, and therefore the absorbent material comprised therein, is directly positioned adjacent to the source of the fluid that has to be absorbed, without relying onto the far less accurate positioning achieved by wearing a panty garment having a disposable absorbent article already applied on it.

Application of a disposable absorbent article directly to the body of the wearer may be particularly advantageous in case of shaped absorbent articles, in order to get an even better body fit by the more precise positioning of the article achievable with a direct application to the body.

Disposable absorbent articles intended to be applied directly to the body usually do not need the traditional rather strong systems for fastening the article to the garment after application to the body, particularly those articles that are shaped to achieve a better body fit, owing to their increased capability of staying in place with preferably no need for a panty fastening system.

A problem with body applied disposable absorbent articles is that the handling/manipulation of the article during at least the first application to the body, and possibly, for articles with no panty fastening system, during subsequent removals and reapplications, until the last removal in order to dispose of it, has to be performed directly on the article itself, while traditional disposable absorbent articles can be more easily handled by means of the panty to which they are initially positioned and adhered, whereby the panty is actually used as a handling aid. This drawback is particularly evident when the article has to be removed from the body when it is dirty, which is almost always the case when the article has to be disposed of, and also for a temporary removal e.g. in case of use of the toilet, or for an intermediate check. The possibly dirty body applied disposable absorbent article has in fact to be touched by the user with her hands, e.g. along the edges where it is presumably still clean, in order to take it off and possibly to put it on again, and moreover has to be kept somewhere while e.g. the user uses the toilet. This of course implies an uncomfortable and unreliable handling of the absorbent article.

Handling aids for disposable absorbent articles intended to be directly applied to the user's body have been described in the prior art, such as for example the string in a tampon. However, the string in a tampon simply provides a link between the user and the product and is typically only used as an aid for the removal of the product, as it does not give any aid for the application of the product, nor a guidance to control its proper placement.

US Patent No. 5,618,282 describes a sanitary napkin adapted to be secured to the wearer's body by means of an adhesive, which also comprises a removal aid for detaching the absorbent article from the body when removal is desired; the removal aid comprises a strip of material longitudinally oriented and secured at its ends to e.g. the garment facing surface of the sanitary napkin, with its medial portion unattached. The strip can therefore be grasped at its medial portion for hygienically removing the sanitary napkin. Although this device provides the body applied sanitary napkin with a device for removing it from the body, it does not constitute any handling aid for applying and/or for properly positioning the sanitary napkin.

In our application entitled "Handling aid for a body applied absorbent article", filed on the same day of the present application, it is described a disposable absorbent article intended for direct application to a user's body, particularly a sanitary napkin, with a handling aid for holding and applying the absorbent article onto the wearer's body, and preferably for its successive removal. More specifically, the disposable absorbent article is provided with a handling aid adapted for the insertion of at least one user's finger for holding and applying the absorbent article. The handling aid is intended to facilitate the right positioning of the article onto the wearer's body, so ensuring a better fit and comfort, particularly in case of disposable absorbent articles that have a tridimensional shape intended to match the surface and the contours of the wearer's body in the pudendal region. The absorbent articles described in the application can also be provided with a tridimensional shape, wherein the handling device contributes to keep said tridimensional shape.

It has now been surprisingly discovered that a handling aid of the type described in the above mentioned application not only makes it easier the handling and the application, and preferably the removal, of disposable absorbent articles intended for direct application to the user's body, moreover helping in finding the right position of the article by using the tactile sensitivity of the fingers, but also the use of such a handling aid allows a better shaping of the disposable absorbent article in contact with the anatomy of the user.

More specifically, the use of said handling aid is capable of achieving a better shape both in combination with disposable absorbent articles that are already shaped before use, and with disposable absorbent articles that can be substantially flat before use, but can be formed into a tridimensional shape by the use of said handling aid for the application of the article to the body.

It is therefore an object of the present invention to provide a use of a handling aid for holding and applying directly to a user's body a disposable absorbent article, which allows the manipulation of the disposable absorbent article into an improved shape when applying it to a position directly on the body.

It is a further object of the present invention to provide a use of such a handling aid that allows the formation of a disposable absorbent article into a tridimensional shape during application to the body, with the disposable absorbent article that is possibly initially flat before use.

### SUMMARY OF THE INVENTION

The present invention refers to a use of a means for holding a disposable absorbent article and for applying directly to a user's body the disposable absorbent article having a body facing surface and a garment facing surface, a longitudinal symmetry plane, and comprising a liquid pervious topsheet, a liquid impervious backsheet joined to the topsheet, and an absorbent element intermediate the topsheet and the backsheet. The means are located on the garment facing surface and being transversely oriented for insertion of at least one finger of a user, or of a person taking care of the user, for holding and applying the absorbent article. The use comprises the steps of:
a) inserting at least one finger in the means for applying the article;
b) manipulating the article with the means into a position directly on the body.

### BRIEF DESCRIPTION OF THE DRAWINGS

While the specification concludes with claims particularly pointing out and distinctly claiming the present invention, it is believed that the present invention will be better understood from the following description in conjunction with the following drawings:
FIG. 1 is a perspective view of one embodiment of a sanitary napkin according to the present invention, seen from the side thereof that faces the wearer in use;
FIG. 2 is a cross-sectional view of the sanitary napkin of FIG 1 on line 2-2;
FIG. 3 is a top view of the sanitary napkin of FIG. 1;
FIG. 4 is a curve taken from an anatomical section of the body of a wearer, which schematically represents the central non linear groove of the female anatomy as seen in lateral direction;
FIGS. 5a, 5b, and 5c are cross-sectional views of the sanitary napkin of FIG. 1 on lines 5a-5a, 5b-5b, and 5c-5c, respectively;
FIG. 6 is a perspective view of the sanitary napkin of FIG. 1, seen from the side that lies remote from the wearer in use;
FIG. 7 is a perspective view of an alternative embodiment of a sanitary napkin according to the present invention, seen from the side that lies remote from the wearer in use;
FIG. 8 is a perspective view of a further alternative embodiment of a sanitary napkin according to the present invention, seen from the side that lies remote from the wearer in use;
FIG. 9 is a perspective view of the sanitary napkin of FIG. 8 after formation into a tridimensional shape by means of the handling aid.

### DETAILED DESCRIPTION OF THE INVENTION

This invention relates to disposable absorbent articles for direct application to the user's body, which exhibit absorbency for bodily fluids, the protection of the user's garments from soiling, and improved physical comfort to the user, which are also easy to produce and to package and which are provided with means for holding the absorbent article and for applying it to the wearer's body, and preferably for successively removing it. The invention particularly relates to the use of said means for holding and applying the article to achieve a better shape of the article while applying it to the body.

The disposable absorbent articles are described below by reference to a sanitary napkin or catamenial. The term "sanitary napkin", as used herein, refers to an article which is worn by females externally of the body and adjacent to the pudendal region and which is intended to absorb and contain the various body fluids which are discharged from the body (e.g., vaginal discharges, menses, and/or urine) and which is intended to be discarded after a single use. It should be understood, however, that the present invention is also applicable to other feminine hygiene or catamenial pads such as pantiliners, or other absorbent articles such as incontinence pads, and the like.

The term "use", as used herein, refers to the period of time that starts when the absorbent article is actually put in contact with the anatomy of the user.

The terms "joined" or "affixed", as used herein, encompasses configurations whereby a first member is directly connected to a second member and configurations whereby a first member is indirectly connected to a second member by connecting the first member to intermediate members which in turn are connected to the second member.

As used herein, the term "pudendal" refers to the externally visible female genitalia and is limited to the labia majora, the labia minora, the clitoris, and the vestibule.

According to the present invention, the means for holding and applying the disposable absorbent articles can be incorporated substantially in any known type of disposable absorbent articles intended to be applied directly to the user's body and to be worn externally. Therefore said means can be comprised in disposable absorbent articles that are e.g. of thin or thick type, rectangular or anatomically shaped in plan view, flat or tridimensionally shaped before use, or also intended to be provided with a tridimensional shape during manipulation of the article with the means for holding and applying it, or further intended to achieve a tridimensional shape during the use, e.g. under the forces acting on the article during wearing.

In a preferred embodiment of the present invention, that will be described hereinbelow, the means for holding and applying the disposable absorbent articles are comprised in a thin disposable absorbent article, more specifically in a sanitary napkin, that is intended for direct application to the user's body, and that has moreover a tridimensional structure before use capable of conforming to the various complex body shapes of the female anatomy comprising non-linear grooves and non-planar surfaces, in order to provide increased body fit and comfort, and reduced leakage.

The use of the means for holding and applying the disposable absorbent article in combination with such a disposable absorbent article having a tridimensional structure before use can result in a better shaping of the article achieved during manipulation and positioning of the article itself on the wearer's body by the means itself.

In another preferred embodiment of the present invention the use of the means for holding and applying the disposable absorbent article will result in the actual tridimensional shaping of an initially flat article.

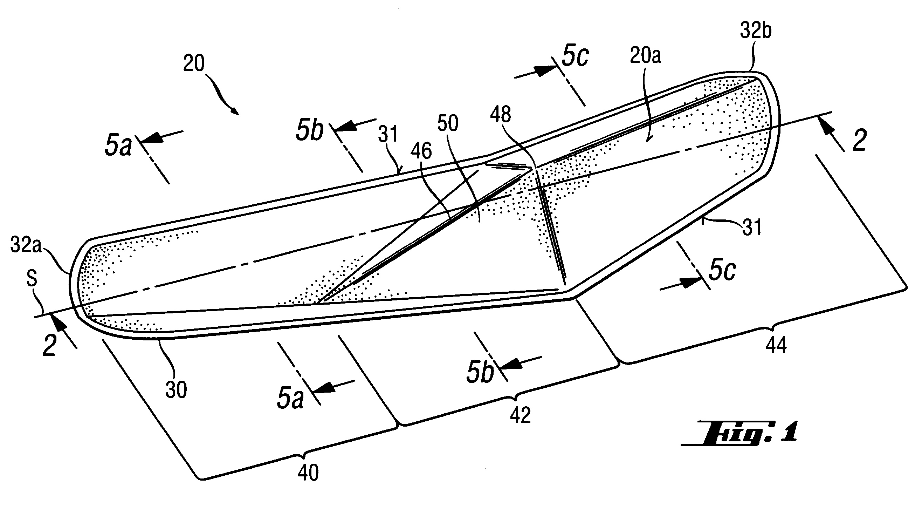

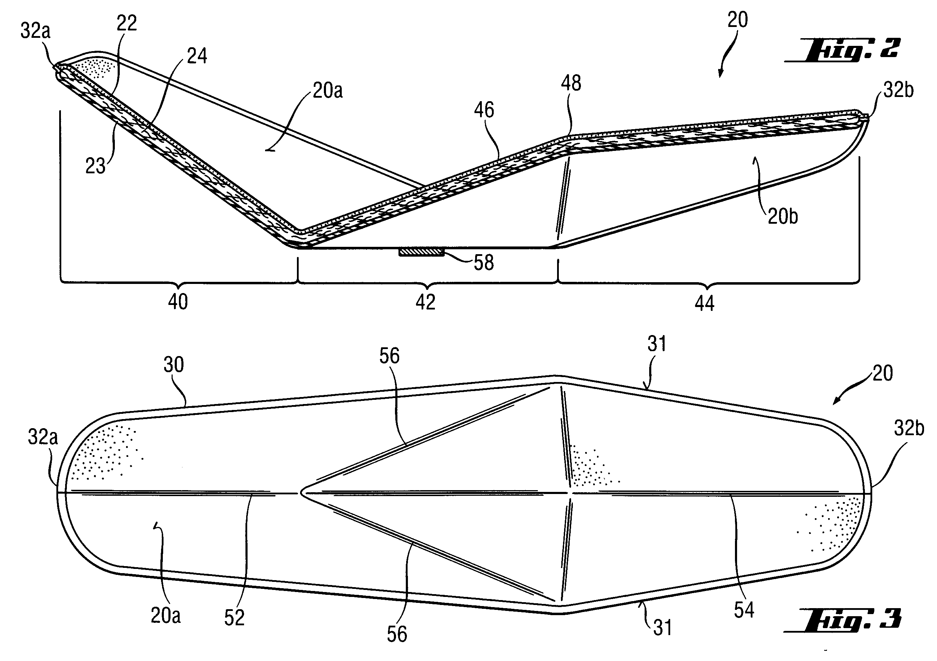

FIG. 1 is a perspective view of a sanitary napkin 20 of the present invention with its preferred tridimensional structure before use, with most of the portion of the sanitary napkin 20 that faces or contacts the wearer, oriented towards the viewer. By saying "before use", it is meant that the preferred sanitary napkin 20 of the present invention is provided with its tridimensional structure before it is actually worn. The sanitary napkin can nevertheless be packaged in a folded flat configuration, being subsequently unfolded to get the tridimensional shape just before wearing it. As better shown in FIG. 2, the sanitary napkin 20 comprises a liquid pervious topsheet 22, a liquid impervious backsheet 23 joined with the topsheet 22, and an absorbent core 24 positioned between the topsheet 22 and the backsheet 23.

The sanitary napkin 20 has two surfaces, a body facing or contacting surface 20a and a garment facing or contacting surface 20b. The body contacting surface 20a is intended to be worn adjacent to the body of the wearer while the garment surface 20b is on the opposite side and is intended to be directed towards the undergarment when the sanitary napkin 20 is worn, e.g. placed against it. Corresponding body facing and garment facing surfaces can also be identified in each single layer that constitutes the sanitary napkin 20, e.g., in the absorbent core 24. The sanitary napkin 20 has a longitudinal symmetry plane S. The term "longitudinal", as used herein, refers to a line, axis or direction in the sanitary napkin 20 that is generally aligned with (e.g., approximately parallel to) a vertical plane which bisects a standing wearer into left and right body halves when the sanitary napkin 20 is worn. The symmetry plane S of the sanitary napkin 20 substantially corresponds to this vertical plane that bisects the standing wearer. While it is preferred that the sanitary napkin 20 is exactly divided by the longitudinal symmetry plane S into two symmetrically equal halves, it is not excluded that the two halves be not specular. The term "transverse", as used herein, refers to a direction or plane that is generally perpendicular to the longitudinal symmetry plane. The term "longitudinally oriented" refers to a direction, as seen in plan view, comprised within ±45 degrees, of the longitudinal symmetry plane S; the term "transversely oriented" similarly refers to any other direction, as seen in plan view.

The terms "front" and "rear", as used herein, refer to portions or edges in the sanitary napkin 20 that are oriented towards the front and rear part of the wearer's body, respectively, when the sanitary napkin 20 is being worn.

The sanitary napkin 20 has a periphery 30, that is defined by the outer edges of the sanitary napkin 20. The longitudinal edges 31 of the sanitary napkin 20 are aligned with the longitudinal symmetry plane S, and the ends edges of the sanitary napkin 20 comprise a front end edge 32a and a rear end edge 32b. The absorbent core 24 of the sanitary napkin has a front portion 40, a central portion 42 and a rear portion 44, each one preferably corresponding to approximately one third of the total length of the absorbent core 24. Corresponding front, central and rear portions can be respectively identified in the sanitary napkin 20 also.

In the preferred embodiment of the present invention the sanitary napkin 20 is tridimensional since it is provided prior to use with a tridimensional structure that is intended to match the complex body shapes of the female anatomy. The tridimensional structure has preferably a structural tridimensionality, by "structural tridimensionality" being meant that the structure cannot be completely flattened onto a flat surface while keeping its integrity, that is, without being in any case e.g. torn, crushed or squeezed. In other words, the tridimensional structure cannot be achieved by simply folding or pleating an initially flat article, but is inherently owned by the absorbent article according to the present invention. The tridimensional sanitary napkin 20 of the present invention has preferably a substantially constant thickness, that is more preferably less than 5 mm; the sanitary napkin can be therefore considered of the thin type.

While the topsheet, the backsheet, and the absorbent core may be assembled in a variety of well known configurations (including so called "tube" products or side flap products), FIG. 1 shows a preferred embodiment of the sanitary napkin 20 in which the topsheet 22 and the backsheet 23 have length and width dimensions generally larger than those of the absorbent core 24. The topsheet 22 and the backsheet 23 extend beyond the edges of the absorbent core 24 to thereby form the periphery 30 of the sanitary napkin 20.

The topsheet 22 is compliant, soft feeling, and non-irritating to the wearer's skin. Further, the topsheet 22 is liquid pervious, permitting liquid (e.g. menses and/or urine) to readily penetrate through its thickness. A suitable topsheet 22 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibres (e.g., wood or cotton fibers), synthetic fibres (e.g., polymeric fibres such as polyester, polypropylene, or polyethylene fibres); or from a combination of natural and synthetic fibres.

A preferred topsheet comprises an apertured formed film. Apertured formed films are preferred for the topsheet because they are pervious to body fluids and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer.

Suitable formed films are described in U.S. Pat. No. 3,929,135, issued to Thompson on December 30, 1975; U.S. Pat. No. 4,324,246, issued to Mullane, et al. on April 13, 1982; U.S. Pat. No. 4,342,314, issued to Radel, et al. on August 3, 1982; U.S. Pat. No. 4,463,045, issued to Ahr, et al. on July 31, 1984; and U.S. Pat. No. 5,006,394, issued to Baird on April 9, 1991. A preferred topsheet for the absorbent article of the present invention is a formed film described in one or more of the above patents and marketed on sanitary napkins by The Procter & Gamble Company of Cincinnati, Ohio as "DRI-WEAVE".

In a preferred embodiment of the present invention, the body or exposed surface of the formed film topsheet is hydrophilic so as to help liquid transfer through the topsheet faster than if the body surface were not hydrophilic so as to diminish the likelihood that menstrual fluid will flow off the topsheet rather than flowing into and being absorbed by the absorbent core. In a preferred embodiment, surfactant is incorporated into the polymeric materials of the formed film topsheet such as is described in U.S. Patent Application Serial No. 07/794,745, Aziz et al., filed on November 19, 1991. Alternatively, the body surface of the topsheet can be made hydrophilic by treating it with a surfactant such as is described in U.S. 4,950,254.

The absorbent core 24 may be any absorbent means that is capable of absorbing or retaining liquids (e.g., menses and/or urine). The absorbent core 24 may be manufactured in a wide variety of sizes and shapes (e.g., rectangular, oval, hourglass, asymmetric, etc.) and from a wide variety of liquid-absorbent materials commonly used in sanitary napkins and other absorbent articles such as comminuted wood pulp that is generally referred to as airfelt. Examples of other suitable absorbent materials include creped cellulose wadding, modified cross-linked cellulose fibres (such as those described in U.S. Patent No. 5,217,445 issued to Young, et al. on June 8, 1993), capillary channel fibres (that is, fibres having intra-fibre capillary channels such as those described in U.S. Patent No. 5,200,248 issued to Thompson, et a. on April 6, 1993), absorbent foams (such as those described in U.S. Patent No. 5,260,345, issued to DesMarais, et al. on November 9, 1993 and U.S. Patent No. 5,268,244 issued to DesMarais, et al. on December 7, 1993), thermally bonded airlaid materials (such as those material described in U.S. Patent Application Serial No. 08/141,156, entitled "Catamenial Absorbent Structures Having Thermally Bonded Layers For Improved Handling of Menstrual Fluids and Their Use In Catamenial Pads Having Improved Fit and Comfort" filed in the name of Richards, et al. on October 21, 1993), absorbent sponges, synthetic staple fibres, polymeric fibres, hydrogel-forming polymer gelling agents, peat moss, tissue including tissue wraps and tissue laminates, or any equivalent materials or combinations of materials. Suitable absorbent cores comprising foams are described in European Applications 0 598 833, 0 598 823 and 0 598 834. Suitable absorbent cores comprising tissue laminates with particles of hydrogel-forming polymer gelling agents comprised therebetween are described in International Patent Applications WO 94/01069 and WO 95/17868.

The configuration and construction of the absorbent core may also be varied (e.g., the absorbent core may have varying caliper zones, e.g., profiled so as to be thicker in the centre), hydrophilic gradients, superabsorbent gradients, or lower density and lower average basis weight acquisition zones; or may comprise one or more layers or structures. The total absorbent capacity of the absorbent core should, however, be compatible with the design leading and the intended use of the sanitary napkin. Further, the size and absorbent capacity of the absorbent core may be varied to accommodate different uses such as incontinence pads, pantiliners, regular sanitary napkins, or overnight sanitary napkins. Preferably the absorbent articles of the present invention are sanitary napkins which are uniform in thickness.

The backsheet 23 and the topsheet 22 are positioned adjacent the garment facing surface 20b and the body facing surface 20a, respectively, of the absorbent core 24 and are preferably joined thereto and to each other by attachment means (not shown) such as those well known in the art. For example, the backsheet 23 and/or the topsheet 22 may be secured to the absorbent core 24 or to each other by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota under the designation HL-1258 or H-2031. The attachment means will preferably comprise an open pattern network of filaments of adhesive as is disclosed in U.S. Patent 4,573,986 entitled "Disposable Waste-Containment Garment", which issued to Minetola, et al. on March 4, 1986. An exemplary attachment means of an open pattern network of filaments comprises several lines of adhesive filaments swirled into a spiral pattern such as illustrated by the apparatus and method shown in U.S. Patent 3,911,173 issued to Sprague, Jr. on October 7, 1975; U.S. Patent 4,785,996 issued to Zieker, et al. on November 22, 1978; and U.S. Patent 4,842,666 issued to Werenicz on June 27, 1989. Alternatively, the attachment means may comprise heat bonds, pressure bonds, ultrasonic bonds, dynamic mechanical bonds, or any other suitable attachment means or combinations of these attachment means as are known in the art.

The backsheet 23 is impervious to liquids (e.g., menses and/or urine) and is preferably manufactured from a thin plastic film, although other flexible liquid impervious materials can also be used. In use, the backsheet 23 is interposed between the absorbent core 24 and the user's undergarments. The function of the backsheet 23 is to prevent exudates which may be expelled from or which inadvertently bypass the absorbent core 24 from contacting and soiling the user's undergarments. The backsheet 23 can thus comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm to about 0.015 mm. Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet 23 is preferably embossed and/or matte finished to provide a more clothlike appearance. Further, the backsheet 23 may permit vapours to escape from the absorbent core 24 (i.e., it can be breathable) while still preventing exudates from passing through the backsheet 23.

As illustrated in FIGS. 1 and 2, the sanitary napkin 20 in its preferred embodiment has before use a tridimensional structure with a longitudinal oriented ridge 50 in the central and rear portions 42, 44 of the absorbent core 24, such that the line of intersection 46 of the longitudinal symmetry plane S with the body facing surface 20a has a slope decreasing rearwardly, i.e. towards the rear end edge 32b, in the central portion 42 and in the rear portion 44 of the absorbent core 24. This can be seen more clearly in FIG. 2, where the longitudinal sectional view of the sanitary napkin 20 shows the line of intersection 46 with its decreasing slope in the central and rear portions 42, 44.

The decreasing slope of said line of intersection 46 can be expressed mathematically if said line of intersection 46 is considered in a Cartesian x-y system lying in the symmetry plane S, wherein the x-axis is defined by the two points of intersection of the longitudinal symmetry plane S with the front end edge 32a and the rear end edge 32b of the sanitary napkin 20, substantially corresponding to the points indicated by numerals 32a and 32b in the cross-section view of the sanitary napkin 20 illustrated in FIG. 2, and wherein the body facing surface 20a faces towards positive y values.

With respect to this system of axes one can form the first derivative of the line of intersection 46. According to the present invention, the first derivative of this line 46 in the longitudinal direction has at least one value that is larger in the central portion 42 of the absorbent core 24 than at least one value in the rear portion 44 of the absorbent core 24. This includes the preferred case, illustrated in FIGS. 1 and 2, where the intersection line 46 is always inclined upward towards the rear end edge 32b with two different slopes in the central portion 42 and in the rear portion 44, and also alternative embodiments in which, e.g., the line of intersection 46 is inclined upward in the central portion 42 and downward in the rear portion 44.

The consecutive values of the first derivative of the line of intersection 46 can decrease continuously towards the rear end edge 32b, implying that the line of intersection 46 has a curved profile with a continuously decreasing slope, or, alternatively, the first derivative can assume different discrete values along the length of the intersection line 46. For example, it can be constant in either the central portion 42, or in the rear portion 44, or in both, the latter being the case of the embodiment illustrated in FIGS. 1 and 2, where the intersection line 46 is formed by two substantially rectilinear portions having constant slopes, with a slope change at a point 48 of the line of intersection 46 positioned where the central portion 42 of the absorbent core 24 merges the rear portion 44.

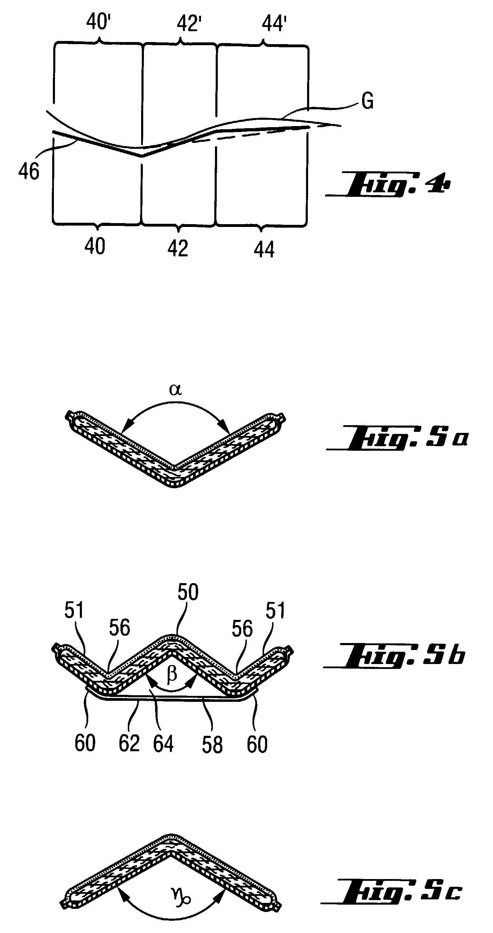

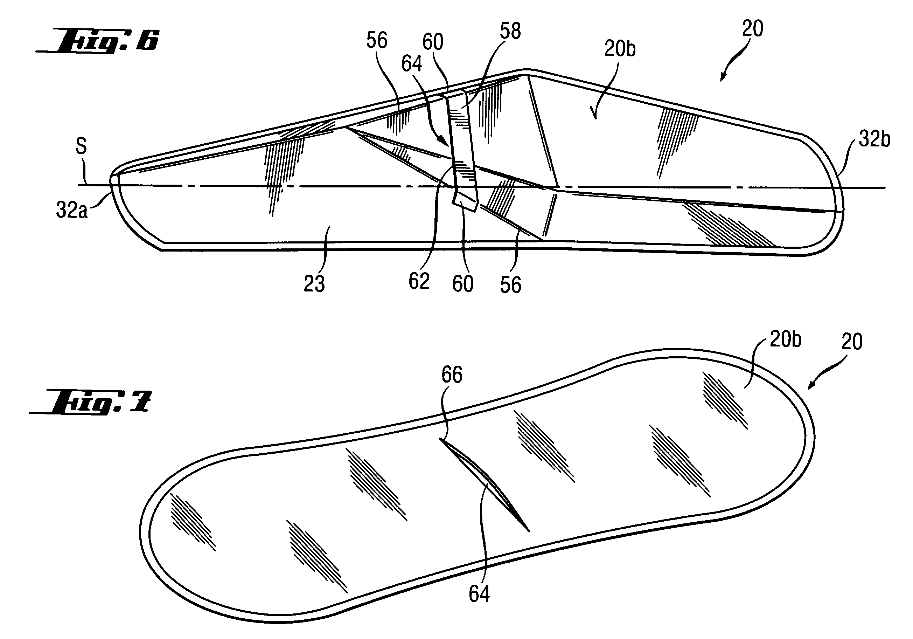

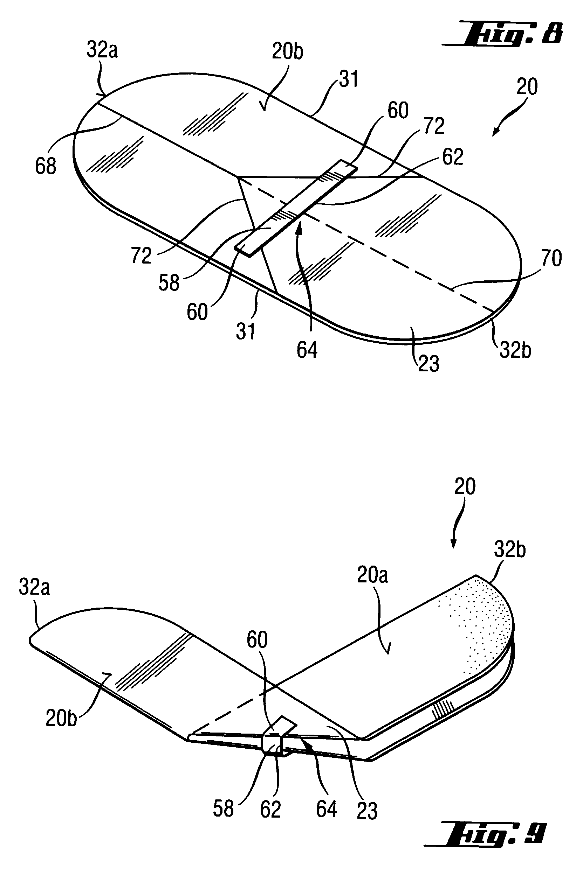

A line of intersection 46 with the above described profile provides the preferred sanitary napkin 20 of the present invention with a longitudinally oriented ridge 50 in the central and rear portions 42, 44 of the absorbent core 24 having a longitudinal non linear profile that is intended to match in use the central non linear groove of the female anatomy extending from the labia majora to the perineum and into the gluteal groove, and having approximately the shape schematically indicated in the corresponding central and rear portions 42', 44' of the curve G illustrated in FIG. 4, where the matching profile of a line of intersection 46 in a sanitary napkin illustrated in FIGS. 1 to 3 is also shown.

The profile of the longitudinally oriented ridge 50 as defined by the line of intersection 46 with its slope decreasing rearwardly can provide the sanitary napkin 20 with an improved fit to the wearer's body. In the preferred embodiment illustrated in FIG. 1, when going from front to rear, the forward portion of the ridge 50, with a substantially constant slope, is intended to fit the groove between the labia majora. The subsequent portion of the ridge 50 that bridges the central and the rear portions 42, 44 of the absorbent core 24, with its change in slope, has a profile that is capable of matching in use the downwardly concave portion of the central non linear groove of the female anatomy in the region going from the rearward part of the labia majora to the perineum, so as to achieve a continuous contact with the body. This provides for a better comfort and for a more effective interception of the fluids as they are released from the body. Finally, the rearward portion of the longitudinally oriented ridge 50, still belonging to the rear portion 44 of the absorbent core 24 and having a constant slope in the embodiment of FIG. 1, is intended to extend between the buttocks, but owing to its slighter slope, as compared to the forward portion of the ridge, it is capable of contacting the body without causing any stress between the anatomy and this portion of the sanitary napkin, which could in turn cause discomfort, and/or prevent the desired substantially continuous contact between the ridge 50 and the wearer's anatomy along the entire length of the non linear groove extending from the labia majora up to the gluteal groove.

In other words, a ridge 50 with a profile having a slope decreasing rearwardly can get further into this non linear groove, as schematically indicated in FIG. 4. The ridge 50 with the profile indicated by the line 46 is in fact capable of following the profile of the groove, indicated by the curve G, by extending past a line, indicated with the dashed line in FIG. 4, that connects two points along the central groove of the body surface where the sanitary napkin has contact with the anatomy, e.g. the two points where the sanitary napkin contacts the body in correspondence of the forward and rearward portions of the ridge. A ridge shaped with a linear profile as those known in the art cannot extend past this line, since such a ridge substantially corresponds to this line, and hence cannot provide a continuous contact with the body along the entire length of the ridge.

In the preferred embodiment of the present invention illustrated in FIGS. 1 and 2 the tridimensional sanitary napkin 20 preferably has a low constant thickness that is less than 5 mm, wherein the tridimensional structure is provided without the use of humps or of regions of different thickness, and it is an inherent feature of the sanitary napkin 20, rather than an added feature, achieved e.g. by bending, folding or joining together an initially planar structure.

As shown in the embodiment of the present invention illustrated in FIGS. 1 and 2, the front portion 40 of the absorbent core 24 is preferably upwardly concave, in order to better conform to the wearer's anatomy in the region of the mons pubis.

The sanitary napkin 20 illustrated in FIGS. 1 and 2 shows a particularly preferred configuration for the front, central and rear portions 40, 42, and 44 of the absorbent core 24. As viewed in transverse section the front, central and rear portions of the absorbent core 24 have respectively a V shape, a W shape, and an inverted V shape, as better shown in FIGS. 5a, 5b, and 5c, where transverse sections of the sanitary napkin 20 taken on lines 5a-5a, 5b-5b, and 5c-5c respectively of FIG. 1 are illustrated.

These different shapes provide the sanitary napkin 20 with the further capability of conforming to the wearer's anatomy in a direction substantially perpendicular to the already defined symmetry plane S. The V shape of the front portion 40 and the inverted V shape of the rear portion 44 merge together gradually in the central portion 42, where the resulting W shape is predisposed to fit the area of the labia majora and of the perineum. In use, the longitudinally oriented ridge 50 is intended to fit the longitudinal central groove as above described, while the side portions 52 bent upwardly can match the groin creases, i.e. the two grooves that are formed between the body and the legs, typically in the area where the panty elastics contact the body.

In the preferred embodiment of the present invention illustrated in FIGS. 1 and 2 the sanitary napkin 20 is provided with an increased capability of conforming to the wearer's anatomy than that simply given by the known differentiated transverse shaping of the different portions of the absorbent core 24.

The tridimensional structure of the sanitary napkin 20 prior to use is such that the width of the angle γ of the inverted V shaped portion increases towards the rear end edge 32b of the sanitary napkin 20 starting from a minimum preferred value at a position corresponding to the merging of the rear portion 44 with the central portion 42 of the absorbent core 24, where it substantially corresponds to the angle β of the central inverted V part of the W shaped central portion 42, which is in turn substantially constant along the entire length of this portion 42. Therefore the rearward portion of the ridge 50, typically positioned in use between the buttocks, can more easily widen its inverted V shape during the wearing of the product without being restrained, so providing the sanitary napkin with a better conformability to the anatomical configuration of the wearer.

A similar feature is preferably provided in the V shaped front portion 40 of the absorbent core 24, where the angle α of the V increases its width towards the front end edge 32a of the sanitary napkin 20 from a minimum preferred value at a point corresponding to the merging of the front portion 40 with the central portion 42. This will allow the portion of the sanitary napkin 20 which is closer to the front end edge 32a to more easily flatten in transverse direction during wearing in order to accommodate the relatively flat front part of the mons pubis, while still providing an overall concave shape to effectively follow the surface of the mons pubis.

The angles of the V shaped front portion 40 and/or of the inverted V shaped rear portion 44 of the absorbent core 24, and consequently of the entire sanitary napkin 20, can therefore increase towards respective end edges 32a and/or 32b up to values around 180°, in order to better accommodate the anatomy of the wearer without inducing any substantial stress in the structure, thus providing for a better fit and comfort.

The preferred feature of the angles increasing towards respective end edges in the V shaped and inverted V shaped portions is achieved by giving the front portion 40 and/or the rear portion 44 of the absorbent core 24 a cup shaped structure with any means known to the man skilled in the art. For example, in the sanitary napkin 20 of the present invention illustrated in FIGS. 1 and 2 this is achieved by cutting away a narrow V shaped portion of material centered along the longitudinal centreline of initially flat front portion 40 and rear portion 44 of the absorbent core 24, and of the topsheet 22 and the backsheet 23 as well, and having substantially the same length of the front portion 40 and of the rear portion 44, and then joining together the cut edges with known means, e.g. by thermobonding, along the junction lines identified as 52 and 54 in FIG. 3. The final tridimensional structure illustrated in FIGS. 1 and 2 is then achieved by suitably bending the non planar sanitary napkin 20, e.g. along lines of preferential bending, formed in the absorbent core 24 by means of e.g. embossments or partial cuts, such as the embossments 56 in FIG. 3, as can be readily determined by the man skilled in the art.

The presence of this preferred feature in the sanitary napkin of the present invention illustrated in FIGS. 1 and 2 can be readily ascertained when folding transversely the sanitary napkin 20 in order to superimpose the front portion 40 or the rear portion 44 of the absorbent core over the central portion 42 along a fold line that approximately in the unfolded sanitary napkin corresponds to a line separating respectively the front portion 40 or the rear portion 44 from the central portion 42: in both cases the folding line will show an angle rather than being rectilinear.

In an alternative embodiment of the present invention a tridimensional shape similar to that illustrated in FIGS. 1 to 5c can also be achieved by comprising in a disposable absorbent article a resilient insert having the desired shape, e.g. between the backsheet and the absorbent core. The insert can be comprised for example only in the central and rear portions of the absorbent article, where the ridge with the desired profile is to be provided, or can extend along the entire length of the absorbent article, in order to provide its whole shape. The resilient insert can be made of any known suitable material, e.g. absorbent or non absorbent material, and can be produced e.g. by thermoforming to get the desired tridimensional shape, preferably with a constant thickness. The insert can completely provide the tridimensional structure, or can alternatively contribute to create and to maintain said structure in an already shaped absorbent article.

The sanitary napkin 20 of the present invention having the preferred embodiment illustrated in FIGS. 1 to 5c and described hereinbefore is means 58 for holding and applying it located on the garment facing surface 20b and being oriented transversely, as illustrated in FIG. 6, that shows a perspective view of the sanitary napkin 20 of FIG. 1 seen from the side that lies remote from the wearer in use, i.e., with the garment facing surface 20b towards the viewer. The means 58 for holding and applying the sanitary napkin 20 are also referred to hereinbelow as a handling aid.

Of course the means 58 for holding and applying the sanitary napkin 20 of the present invention are also intended for use by a person taking care of a user, e.g. a nurse, who handles the sanitary napkin 20 and applies it to the user's body.

In the preferred embodiment of FIG. 6 the means 58 for holding and applying the sanitary napkin 20 comprises an elongated strip of elastic film material 58 oriented perpendicularly to the longitudinal symmetry plane S and located on the garment facing surface 20b of the sanitary napkin 20, in correspondence of the central portion 42 of the absorbent core 24, at a position approximately longitudinally intermediate between the front end edge 32a and the rear end edge 32b of the sanitary napkin 20. The strip 58 is affixed to the backsheet 23 at its two spaced apart ends 60 disposed on opposite sides of the symmetry plane S, with an intermediate portion 62 being not joined to said garment facing surface 20b and defining a space 64, as better shown in FIG. 5c, intended for the insertion of at least one user's finger for holding and applying the sanitary napkin 20. In the embodiment illustrated in FIG. 6, where the sanitary napkin 20 has the preferred tridimensional shape before use, the space 64 is actually comprised between the intermediate portion 62 of the strip 58 and the garment facing surface 32b of the central portion of the sanitary napkin, which is concave on its garment facing surface 20b, since it corresponds to the ridge 50 on the body facing surface 32a. Typically the spaced apart ends 60 of the strip 58 are fixed with known means, e.g., with an adhesive, or by thermobonding, to the garment facing surface 20b of the backsheet 23 at intermediate locations between each bend line corresponding to the embossments 56, and the respective longitudinal edge 31, as can also be seen in FIG. 5b.

The user can put the sanitary napkin 20 on the palm of her hand with the garment facing surface 20b contacting the hand and with the front end edge 32a facing towards the wrist, at the same time inserting typically one of her fingers, e.g. the middle finger, in the space 64 between the intermediate portion 62 of the strip 58 and the backsheet 23. The user can therefore hold the sanitary napkin 20 in her open hand without exerting any force, also owing to the elasticity of the preferred material that constitutes the strip 58, with substantially the front portion of the sanitary napkin 20 lying on her palm. Application to the body can then be easily performed by the user with a single movement of her open hand, which is simple and self-explanatory as putting the empty hand on the body.

Moreover, the movements of the hand and of the fingers allow the user to completely control the manipulation of the sanitary napkin 20 during its application to the body, making use of the tactile sensitivity of the fingers to find the right position for the sanitary napkin 20. Particularly, in the preferred embodiment of the present invention, the finger inserted in the space 64 is substantially aligned with the ridge 50 on the body facing surface 20b of the sanitary napkin 20, and therefore can provide guidance to control the proper placement of the napkin 20 on the body anatomy, i.e. with the ridge 50 suitably registered with the longitudinal non-linear groove of the female anatomy extending from the labia majora to the gluteal groove. The forward portion of the ridge can be e.g. easily identified by the user with her finger inserted in the space 64, and used as a reference to direct the sanitary napkin into an optimal position on the body.

The user can manipulate the sanitary napkin 20 by means of the handling aid while positioning it directly to the body, in order to achieve a better shaping of the sanitary napkin itself upon contact with the body, therefore taking advantage of the adapting capacity of the preferred tridimensional structure of the sanitary napkin that can slightly modify its structure to conform to the wearer's anatomy constituted by the non-linear grooves and non-planar surfaces already described, e.g. by differently bending along its multiple bend lines. This manipulation and adaptation of the sanitary napkin held by means of the handling aid is preferably performed by the user while putting the sanitary napkin into a position directly on her body, by means of a combined action of her hand holding the sanitary napkin and actively manipulating it, and of the body surface contacted by the sanitary napkin itself, to which the napkin is caused to conform and fit. The manipulation and adaptation of the sanitary napkin can be also partially initiated by the user with her hands only before actually putting the sanitary napkin directly on the body, taking advantage of the preferred way in which the sanitary napkin can be held with one hand by means of the handling aid.

The handling aid constituted by the strip 58 also allows an easy removal of the hand once the sanitary napkin 20 is in place, without disturbing or modifying the position of the napkin 20.

Since in the preferred embodiment of the present invention described so far the tridimensional sanitary napkin 20 does not comprise a panty fastening system, the handling aid of the present invention illustrated in FIGS. 5b and 6 preferably also allows an easy removal and, possibly, a subsequent reapplication of the sanitary napkin 20 from the body in order to use the toilet, or to make a check of the product, or in any case in order to finally dispose of the product. The user can in fact easily grab the sanitary napkin 20 while it is being worn by positioning her hand substantially in the same way as for the application, with one of her fingers inserted in the space 64 between the not joined portion 62 of the strip 58 and the backsheet 23. The sanitary napkin 20 can therefore be taken off the body and securely held by the user; the handling aid may also be used to temporarily store the sanitary napkin, e.g. while using the toilet, on the user's hand, with no need for actually holding it with the fingers, or for exerting any force on it.

The handling aid constituted by the strip 58 allows in any event the user to handle/manipulate the sanitary napkin 20 by contacting its garment facing surface 20b only, therefore protecting her hand from the possibly dirty body facing surface 20a.

In the preferred absorbent articles having a tridimensional shape before the use, such as the sanitary napkin 20 in the preferred embodiment described hereinbefore, the handling aid preferably also contributes to keep the tridimensional shape of the article during the use, e.g. in case of body movements that can disturb the proper fit of the product, or when in general there is a risk of collapse of the body fitting tridimensional shape. Otherwise the handling aid, e.g. constituted by the strip 58 illustrated in FIGS. 5b and 6, stays aligned or folded or loose on the garment facing surface 20b of the product and does not disturb the product performance.

In alternative embodiments of the present invention the handling aid can be constituted by more than one strip of material, or by one or more strings, while the material can be also non elastic. The handling aid can be also constituted by a strip arranged as a loop and applied to the garment facing surface 20b of the article, or by a series of loops, intended to allow the insertion of at least one users finger.

The handling aid can also be activated by the user, e.g. by being applied to the garment facing surface of the absorbent article just before use; alternatively, a handling aid e.g. constituted by a strip 58 can be detached e.g. at one of its ends from the garment facing surface of the absorbent article and then repositioned at a different place, in order to e.g. partially control or adapt a tridimensional shape already provided in the absorbent article, or to modify the space 64 available for the insertion of at least one user's finger. A handling aid preferably constituted by a strip 58 could therefore be resealably attached to the garment facing surface 20b of the absorbent article, at either one or both ends 62, e.g. by means of a resealable adhesive, or of a mechanical fastener of the hook and loop type, such as that marketed under the tradename VELCRO. A handling aid in form of a loop could be modified by the user in order to change the diameter of the loop, and hence the space available for the insertion of the finger(s).

In a further alternative embodiment of the present invention the disposable absorbent article can comprise a release cover releasably attached to the garment facing surface of the absorbent article, wherein the handling aid is located on said release cover. In use, after application of the absorbent article to the body by means of the handling aid, the release cover can be detached from the garment facing surface of the article, leaving the adhesive exposed, that can thus serve as a panty fastening adhesive as it is already known in the art. Successive removal of the absorbent article would be performed e.g. with the known method, using the panty, with the now attached absorbent article, as an handling aid.

Alternatively, in a less preferred embodiment of the present invention the absorbent article can be also provided with a panty fastening means, which provides means to attach the article to the undergarment after it has been applied to the body. This would subsequently allow removal of the article from the body in a rather traditional way, i.e. by means of the panty to which the article is adhered. Panty fastening means could be located on a limited portion of the garment facing surface of the absorbent article, in order to avoid the risk of sticking to the user's hand during handling and application of the absorbent article, or, alternatively, it could be activated by the user after the absorbent article has been actually applied to the body, e.g. by removing a release paper. In any case the panty fastening means may comprise a mechanical fastener such as hook and loop fasteners such as marketed under the tradename VELCRO, snaps or holders, which would have the advantage that they do not stick to the user's hand. Alternatively, the absorbent article could be fastened to the undergarment by means of panty fastening adhesive on the backsheet 23. The panty fastening adhesive would provide a means for securing the absorbent article to the panty and preferably a means for securing the absorbent article when soiled to the fold and wrap package for convenient disposal. Any adhesive or glue used in the art for such purposes can be used for the panty fastening adhesive herein. Pressure sensitive adhesives are most preferred. Suitable adhesives include Century A-305-IV manufactured by the Century Adhesives Corporation of Columbus, Ohio, and Instant LOK 34-2823 manufactured by the National Starch and Chemical Company of Bridgewater, New Jersey, 3 Sigma 3153 manufactured by 3 Sigma and Fuller H-2238ZP manufactured by the H.B. Fuller Co.

The panty fastening adhesive can be typically applied to the backsheet by slot coating or spraying in various distribution patterns, such as e.g. continuous or discontinuous strips, intermittent dots, random patterns spirals.

The panty fastening adhesive should be typically covered with a removable release paper or film in order to prevent the adhesive from drying out or adhering to another surface other than the panty. Any commercially available release paper or film may be used. Suitable examples include BL 30MG-A SILOX EI/O and BL 30 MG-A SILOX 4 P/O available from Akrosil Corporation.

In another alternative embodiment of the present invention the handling aid can be also comprised in an initially flat absorbent article, as it is illustrated in FIG. 7, where an alternate type of handling aid is also shown, being constituted by a transversely oriented cut 66 in the garment facing surface 20b of a flat sanitary napkin 20, defining a space 64 for the insertion of at least one user's finger for holding and applying, and preferably removing, the absorbent article 20, in a way substantially equivalent to that already described for the preferred tridimensional absorbent article

A handling aid according to the present invention is particularly advantageous in combination with a flat absorbent article intended for direct application to the body, since the handling aid not only makes the handling/application and preferably the removal of the article easier, but also provides the user with an indication on how to hold the article and how to position it on the body, which otherwise is typically missing in a flat absorbent article.

In this case the use of the handling aid can also form the initially flat sanitary napkin 20 into a tridimensional shape that fits the body anatomy while applying the napkin to the body, by manipulating the sanitary napkin 20 and causing it to adapt to the body by means of the handling aid.

In a further preferred embodiment of the present invention the use of the means for holding and applying the absorbent article can be considered in combination with an e.g. initially flat disposable absorbent article comprising lines of preferential bending intended to assist the formation of the article in a preferred tridimensional shape during manipulation and application of the article to the wearer's body by means of the handling aid.

FIGS. 8 and 9 schematically illustrate a sanitary napkin 20 similar to those described in the International Patent Application WO 9602217, comprising means 58 for holding and applying the napkin to the user's body. In FIG. 8 a perspective view of a sanitary napkin 20 in a flat state before use is shown, with the garment facing surface 20b oriented towards the viewer. The sanitary napkin 20 comprises bend lines to assist the formation of a preferred tridimensional structure by the use of the means 58 for holding and applying the sanitary napkin to the wearer's body. In the embodiment of FIG. 8 a main front bend line 68 begins at the front end edge 32a and extends to about the beginning of a rear main bend line 70, both front and rear bend lines being substantially aligned with the symmetry plane S of the sanitary napkin 20; two rearwardly diverging bend lines 72 also begin at about the beginning point of the main rear bend line 70, each extending towards the rear end edge 32b up to the respective longitudinal edge 31. The means 58 for holding and applying the sanitary napkin 20 comprises an elongated strip 58 of a thin film material oriented perpendicularly to the longitudinal symmetry plane S and located on the garment facing surface 20b, at a position approximately longitudinally intermediate between the front end edge 32a and the rear end edge 32b. The strip 58 is affixed to the backsheet 23 at its two spaced apart ends 60 disposed on opposite sides of the symmetry plane S, with an intermediate portion 62 not joined to the garment facing surface 20b and defining a space 64 intended for the insertion of at least one user's finger for holding and applying the sanitary napkin 20.

In order to use the holding and applying means 58 to apply the sanitary napkin 20 to the wearer's body, and to form at the same time the sanitary napkin 20 into a preferred tridimensional shape intended to conform and fit the body anatomy, the user puts the flat sanitary napkin 20 on the palm of her hand with the garment facing surface 20b contacting her hand and with the front end edge 32a towards her wrist, at the same time inserting e.g. her middle finger in the space 64 where the strip 58 is not affixed to the backsheet 23. The user can now hold the sanitary napkin 20 in her open hand, and with a single movement of the hand can manipulate it into a position directly on the body, as already explained with respect to the embodiment illustrated in FIGS. 1 to 6, at the same time forming the sanitary napkin 20 into the preferred tridimensional shape illustrated in FIG. 9, while applying it to the body. This formation is assisted by the bend lines 68, 70, 72 comprised in the sanitary napkin structure, that are intended to preferentially bend upwards and downwards to form the tridimensional shape of FIG. 9, where the handling aid 58 is shown slightly loose in its intermediate portion 62. The user can in fact use her middle finger, substantially aligned with the symmetry plane S, to create the upwardly convex shape of the rear portion of the sanitary napkin 20 along the main rear bend line 70, and the whole hand to form the upwardly concave shape of the front portion of the sanitary napkin 20. The terms "front portion" and "rear portion" have a meaning that is similar to that related to the embodiment of FIGS. 1 and 2.

The tridimensional shape shown in FIG. 9 is in fact somewhat similar to that of the preferred embodiment of the present invention illustrated in FIGS. 1 to 6, but without the preferred non linear upwardly convex profile of the rear portion of the napkin, and without the particularly cup shaped front portion. Although maybe less preferred than the embodiment of FIGS. 1 to 6 from the point of view of body fitting and conformability to the anatomy, this embodiment shows a preferred use of the means for holding and applying an absorbent article according to the present invention and intended for both application and shaping of the article.

The preferred shaping of the initially flat sanitary napkin 20 into the tridimensional structure is preferably performed during the application of the sanitary napkin 20 by means of the handling aid, by manipulating the sanitary napkin 20 with the hand against the wearer's body anatomy.

The absorbent articles of the present invention, particularly the sanitary napkin 20, have a length that preferably ranges among the typical values commonly used for different sizes of said sanitary articles intended for substantially external disposition adjacent to the body of the wearer. Particularly, the central and rear portions 42 and 44 of the absorbent core 24 do not have preferably a length which is smaller than the total maximum length of the labia majora of an average user.

The handling aid of the present invention does not necessarily extend across the entire width of the absorbent article, in order to define a suitable space for the insertion of at least one user's finger, which is capable of achieving a sufficiently firm fit with said at least one finger.

As illustrated in the preferred embodiment of FIG. 6, the handling aid does not extend in longitudinal direction over a major portion of the length of the disposable absorbent article; preferably, it extends over less than 10% of said length, being more preferably a narrow strip with a width, extending in said longitudinal direction, of about 1 cm.

The absorbent article of the present invention may further comprise an odour-control material for controlling unpleasant odours associated with absorbed body fluids.

Any known odour-control agent or any combination thereof that can be suitably included in a disposable absorbent article, including other materials such as binders and/or substrates, can be comprised in the absorbent article of the present invention as the odour-control material.

The odour-control material can be incorporated into the absorbent article by methods known in the art, for example layered on or into the absorbent core or mixed within the absorbent core.

In an alternate embodiment of the present invention, the absorbent article comprising means for holding and applying the article directly to the wearer's body may have two flaps (not shown), each of which is adjacent to and extends laterally from the respective side edge of the absorbent core. The flaps are configured to drape over the edges of the wearer's panties in the crotch region so that the flaps are disposed between the edges of the wearer's panties and the wearer's thighs. The flaps help serve to prevent soiling of the wearer's body and panties by menstrual fluid, preferably by forming a double wall barrier along the edges of the panty. The flaps may be also provided with attachment means on their garment facing surface so that the flaps can be folded back under the panty and attached to the garment facing side of the panty.

The flaps may be constructed of various materials including materials used for the topsheet 22, backsheet 23, combinations thereof, and may be a laminate having tissue in the centre. Further, the flaps may be a separate element attached to the main body of the tridimensional absorbent article or can comprise extensions of the topsheet 22 and/or backsheet 23. It is recommended, however, that the flaps have a liquid impervious backsheet to prevent body fluids which reach the flaps from soiling the edges of the wearer's panties.

Preferred flaps that are suitable or adaptable to the tridimensional absorbent article of the present invention are disclosed in U.S. Pat. No. 4,687,478 issued to Van Tilburg on Aug. 18, 1987; U.S. Pat. No. 4,589,876 issued to Van Tilburg on May 20, 1986; and U.S. Pat No. 4,608,047 issued to Mattingly on Aug. 26, 1986.

Optionally, but preferably over the above mentioned flaps, the tridimensional absorbent article may comprise components that naturally wrap the sides of a wearer's panties. Sanitary napkins having components that naturally wrap the sides of a wearers panties suitable for use with the tridimensional absorbent article of the present invention are disclosed in U.S. Patent Application Serial No. 08/096,121 entitled "Absorbent Article having Panty Covering Components that Naturally Wrap the Sides of Panties", filed July 22, 1993, in the names of Lavash, et al and U.S. Patent Application Serial No. 08/277733 entitled "Absorbent Articles Having Undergarment Covering Components with Zones of Extensibility", filed July 20, 1994, in the names of Weinberger, et al.

In further alternate embodiments of the present invention the absorbent article can also comprise additional elements, such as an acquisition layer or a secondary topsheet positioned between the topsheet 22 and the absorbent core 24 or, alternatively, in any other suitable position.

Although the disposable absorbent article of the present invention has been described with reference to a sanitary napkin, it can be used beneficially in the context of other disposable absorbent articles such as panty liners and incontinence articles. The disposable absorbent article may thus also have all those features and parts which are typical for products in the context of their intended use.

## Claims

1. Use of a means for holding a disposable absorbent article and for applying said disposable absorbent article directly to a user's body, said disposable absorbent article having a body facing surface and a garment facing surface, a longitudinal symmetry plane, and comprising a liquid pervious topsheet, a liquid impervious backsheet joined to said topsheet, and an absorbent element intermediate said topsheet and said backsheet, said means being located on said garment facing surface and being transversely oriented for insertion of at least one finger for holding and applying said absorbent article, said use comprising the steps of:
a) inserting at least one finger in said means for applying said article;
b) manipulating said article with said means into a position directly on the body.

2. Use according to claim 1, characterized in that it further comprises the step of forming said article into a tridimensional shape while applying said article to the body.

3. Use according to any of claims 1 or 2, wherein said means comprises at least one elongate piece of material joined at two spaced apart ends to said garment facing surface and further comprising an intermediate portion not joined to said garment facing surface.

4. Use according to any preceding claim, wherein said means comprises an elongate strip of material.

5. Use according to claim 3, wherein said spaced apart ends are disposed on opposite sides of said longitudinal symmetry plane.

6. Use according to claim 2, wherein said article is substantially flat prior to said manipulation of said article with said means.

7. Use according to claim 2, wherein said article is provided with bend lines to assist said forming of said article into said tridimensional shape.
